# EUROPEAN PATENT APPLICATION

(11) **EP 4 640 825 A1**
(43) Date of publication of application: **29.10.2025**
(21) Application number: 25167947.8
(22) Date of filing: 02.04.2025
(51) Int. Cl.: C12N 5/071

(54) **METHOD FOR GENERATION OF STEM CELLS IN CULTURED LIVER TISSUE**

(30) Priority: 03.04.2024 IT 202400007321
(71) Applicant: UNIVERSITA' DEGLI STUDI DI TRIESTE, 34127 Trieste (IT)
(72) Inventor: SORRENTINO, Giovanni, 34127 TRIESTE (IT); ANFUSO, Beatrice, 34126 TRIESTE (IT)
(74) Representative: Bianchetti & Minoja with Trevisan & Cuonzo IPS SRL

(57) **Abstract**

The subject of the present invention is an *in vitro* method for generation of bipotent liver stem cells in liver tissue sections in PCLS (Precision-cut Liver Slice) form in culture. A further subject of the invention is a culture medium for PCLS of human or mouse origin and the use thereof to stimulate liver stem-cell generation in liver tissue cultures.

## Description

The present invention relates to an *in vitro* method for generation of bipotent liver stem cells in cultured liver tissue. The method according to the invention advantageously applies to screening of medicaments or molecules involved in the diagnosis, treatment or prevention of liver disease. A further subject of the invention is a culture medium for PCLSs (precision-cut liver slices) of human or mouse origin.

### BACKGROUND TO THE INVENTION

Chronic degenerative liver diseases are a set of progressive disorders characterised by death of the liver cells, called hepatocytes, an event that prejudices the functionality of the organ with time. The most common diseases are cirrhosis of the liver and chronic hepatitis. Cirrhosis of the liver is characterised by the formation of fibrous scars in the liver tissue, caused by repeated lesions over time. Said process adversely affects the normal structure of the organ, impeding its ability to perform vital functions such as protein production and metabolisation of chemical substances. Chronic hepatitis involves long-term liver inflammation, often triggered by persistent viral infections or chronic metabolic disorders. Both conditions can give rise to serious complications, such as liver failure, hepatocellular carcinoma and portal hypertension.

Bipotent liver stem cells are a specific type of adult stem cells present in the liver, which can differentiate into two main cell lines: hepatocytes (functional liver cells) and bile duct epithelial cells (cholangiocytes) (involved in bile duct formation). Said cells form a multipotent stem-cell population that plays a crucial part in the liver regeneration and repair processes, especially during chronic liver disease (Köhn-Gaone, J., Gogoi-Tiwari, J., Ramm, G. A., Olynyk, J. K. & Tirnitz-Parker, J. E. E. The role of liver progenitor cells during liver regeneration, fibrogenesis, and carcinogenesis. American Journal of Physiology-Gastrointestinal and Liver Physiology 310, G143-G154 (2016)).

The activation process of bipotent stem cells involves dedifferentiation of bile duct epithelial cells into bipotent adult stem cells able to proliferate and coordinate a damage repair response ("wound healing"). Said process is called "ductular reaction" (DR) ( Popper, H., Kent, G. & Stein, R. Ductular cell reaction in the liver in hepatic injury. J Mt Sinai Hosp N Y 24, 551-556 (1957)).

*In vitro* study of activation of the bipotent liver stem cells, and therefore of DR, is highly complex, because it is controlled both by endogenous processes of the stem cells and by their interaction with the physicochemical microenvironment (extracellular matrix) and cellular microenvironment (physical and functional interaction with hepatocytes, stellate cells, Kupffer cells and others). The study of said process therefore requires the physiological and histological characteristics of the original tissue to be maintained *in vitro.* Some of the methodologies most commonly used to date to study bipotent liver stem cells *in vitro* include two-dimensional (2D) cell cultures, three-dimensional (3D) cell cultures, liver organoids with 3D structures originating from adult stem cells not transformed into natural or synthetic matrices, tissue engineering or cell cocultures. However, said techniques involve a number of drawbacks. Firstly, they introduce a great deal of variability between samples, and in some cases are characterised by a very high level of technical complexity. Moreover, none of said techniques accurately simulates the liver microenvironment.

The precision-cut liver slice (PCLS) technique (Olinga, P. & Schuppan, D. Precision-cut liver slices: a tool to model the liver ex vivo. J Hepatol 58, 1252-1253 (2013)) is by far the most accurate of the most physiologically significant systems able to reproduce the liver microenvironment. It consists of thin liver-tissue slices obtained by a very precise cutting procedure, used in various research contexts.

The main advantage of said system is that the technique maintains the three-dimensional structure and histological cell organisation of the native liver tissue. Moreover, PCLSs contain all the cells, both parenchymal and non-parenchymal, present in adult liver tissue.

Although PCLSs are used to study liver physiology, their application in the study of bipotent stem cells in the liver remains unexplored, because PCLSs do not contain liver stem cells. To date, no methods have been devised which allow the appearance and activation of liver stem cells in PCLSs.

### STATE OF THE ART

Huch, M. et al. ("In vitro expansion of single Lgr5+ liver stem cells induced by Wnt-driven regeneration", Nature 494, 247-250 (2013)) describes a protocol for keeping alive mouse liver stem cells, isolated in culture, using a medium containing various growth factors.

Huch, M. et al. (Long-Term Culture of Genome-Stable Bipotent Stem Cells from Adult Human Liver. Cell 160, 299-312 (2015)) describes a protocol for keeping alive human liver stem cells, isolated in culture, using a medium containing various growth factors.

De Graaf, I. A. M. et al. ("Preparation and incubation of precision-cut liver and intestinal slices for application in drug metabolism and toxicity studies." Nat Protoc 5, 1540-1551 - 2010) describes a protocol for the production and maintenance in culture of PCLSs under physiological conditions and in the absence of stem cells.

### DESCRIPTION OF FIGURES

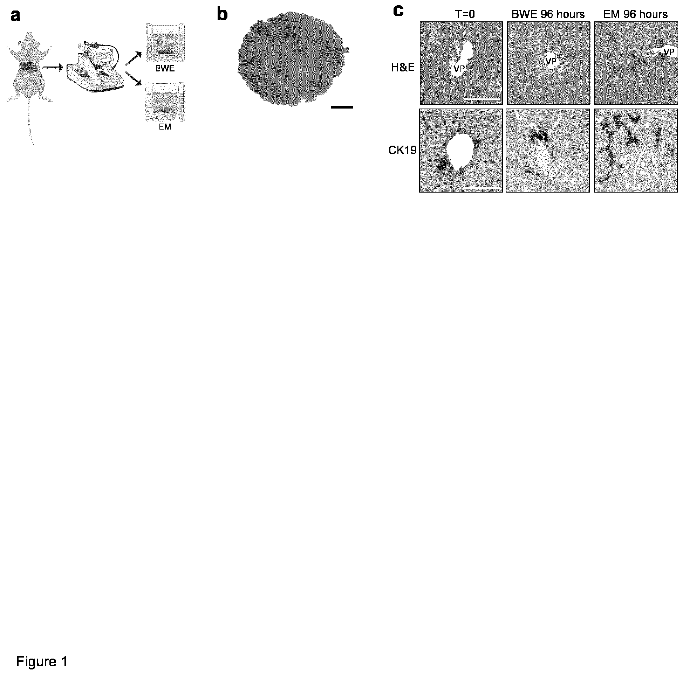

**Figure 1****:** scheme of mouse PCLS preparation procedure and ductular reaction analysis. **a)** PCLSs were prepared from mouse liver using a vibratome, embedded in a reconstituted basement membrane, and maintained in culture for 48 or 96 hours. The medium implemented (expansion medium: EM) was compared with the standard culture conditions (Basal Williams' E: BWE). **b)** Macroscopic appearance of PCLSs. Scale bar: 1 mm. **c)** Staining of PCLSs with haematoxylin-eosin (H&E, top line) and immunohistochemical staining for liver stem-cell marker CK19 (bottom line), under the various experimental conditions. PV: portal vein. Scale bar: 100 µm.

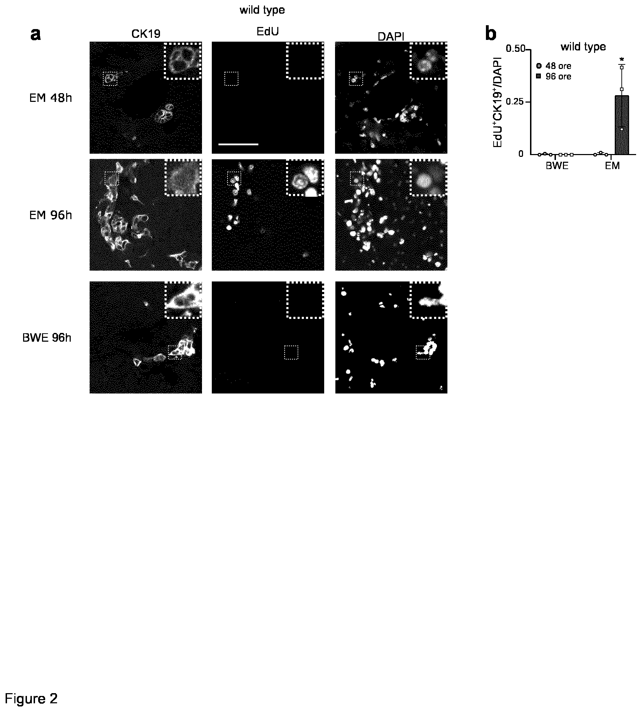

**Figure 2****:** monitoring of stem-cell proliferation (with EdU assay) in mouse PCLSs. **a)** Immunofluorescence for Ck19 (left), EdU (centre) and DAPI (right) in PCLSs obtained from wild-type mice. Scale bar: 50 µm. **b)** Quantitation of cells positive for both CK19 and EdU in PCLSs maintained in culture for 48 or 96 hours in BWE or EM (n=3). The data are presented as mean ± SD; *P < 0.05, two-way ANOVA.

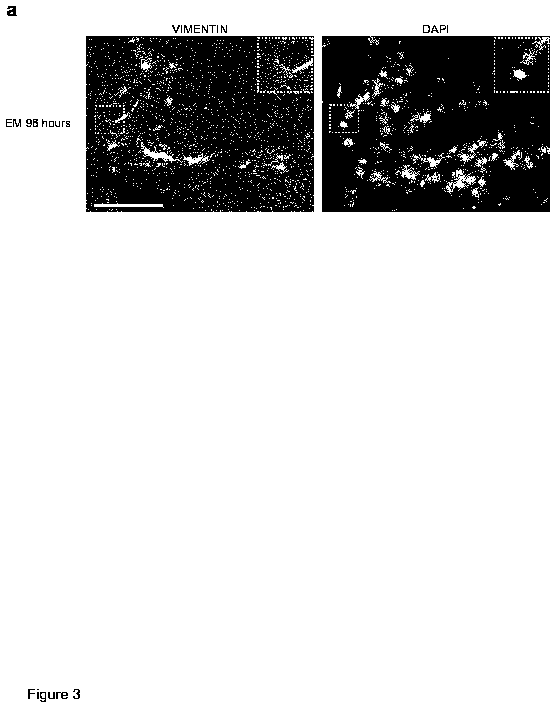

**Figure 3****:** presence of mesenchymal cells in mouse PCLSs. Immunofluorescence of vimentin close to portal areas. Scale bar: 50 µm.

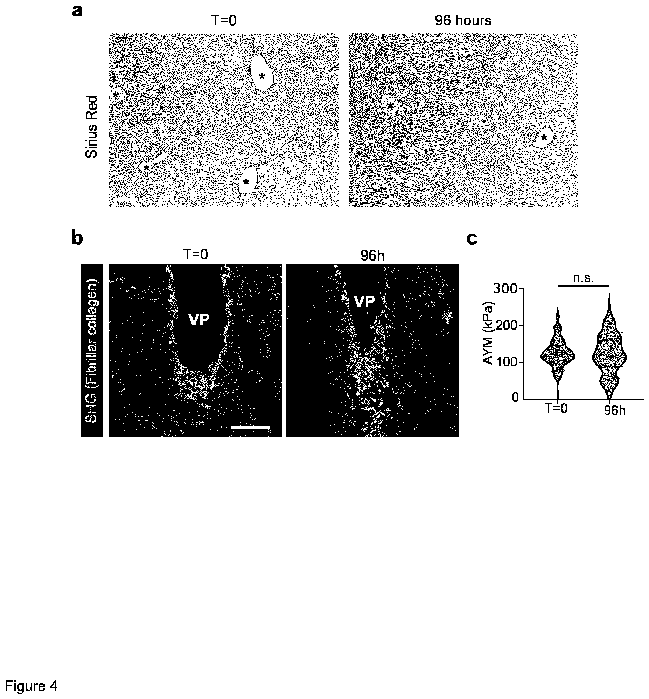

**Figure 4****:** mechanical microenvironment in mouse PCLSs. **a)** Staining of collagen with Sirius Red in PCLSs. Scale bar: 50 µm. **b)** Distribution of collagen fibrils in portal areas of PCLSs observed by second harmonic generation (SHG) microscopy. Scale bar: 50 µm. **c)** Quantitation of apparent Young's modulus of elasticity (AYM) in the areas of the PCLSs enriched with collagen by atomic-force microscopy. n.s.: not significant, Student's "t" test.

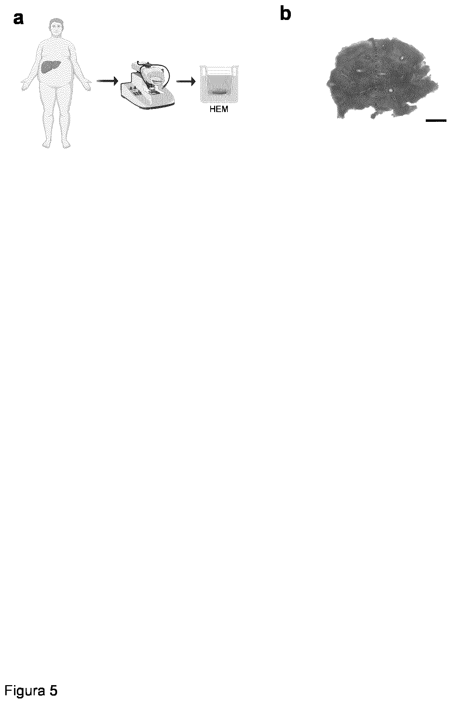

**Figure 5****:** scheme of procedure for preparation of human PCLSs. **a)** The PCLSs were prepared from human liver samples, embedded in a reconstituted basement membrane and maintained in culture for 8 days. The medium implemented (EM) was compared with the standard culture conditions (BWE). **b)** Macroscopic appearance of PCLSs. Scale bar: 1 mm.

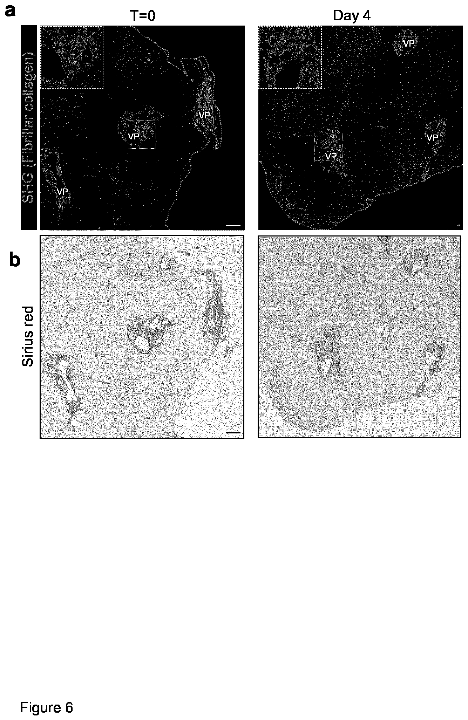

**Figure 6****:** mechanical microenvironment in human PCLSs. **b)** Distribution of collagen fibrils in portal areas of PCLSs observed by second harmonic generation (SHG) microscopy. Scale bar: 100 µm. **b)** Staining of collagen with Sirius Red in PCLSs. Scale bar: 100 µm.

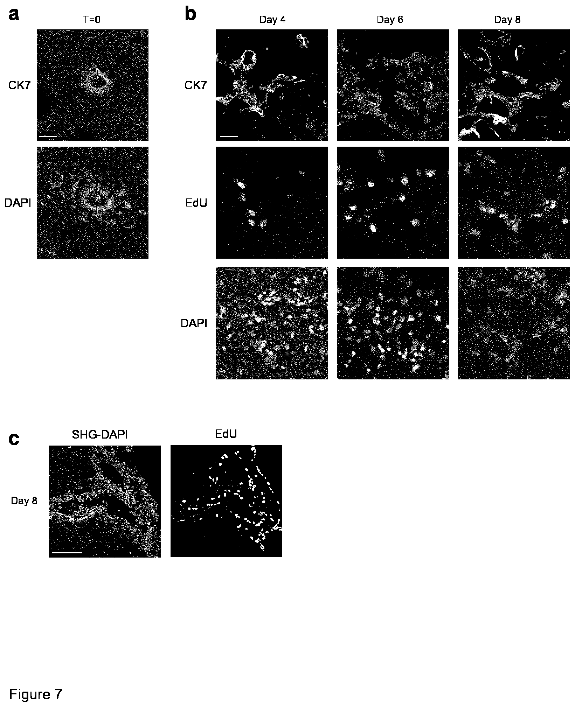

**Figure 7****:** monitoring of stem-cell proliferation (with EdU assay) in human PCLSs. **a)** Immunofluorescence of CK7 (top) and DAPI (bottom) in PCLSs obtained from human liver biopsies. **b).** Immunofluorescence for CK7 (top), EdU (centre) and DAPI (bottom). **c)** Distribution of collagen fibrils in human PCLSs observed by second-generation harmonic microscopy (left) and of proliferation by EdU assay (right). Scale bar: 50 µm.

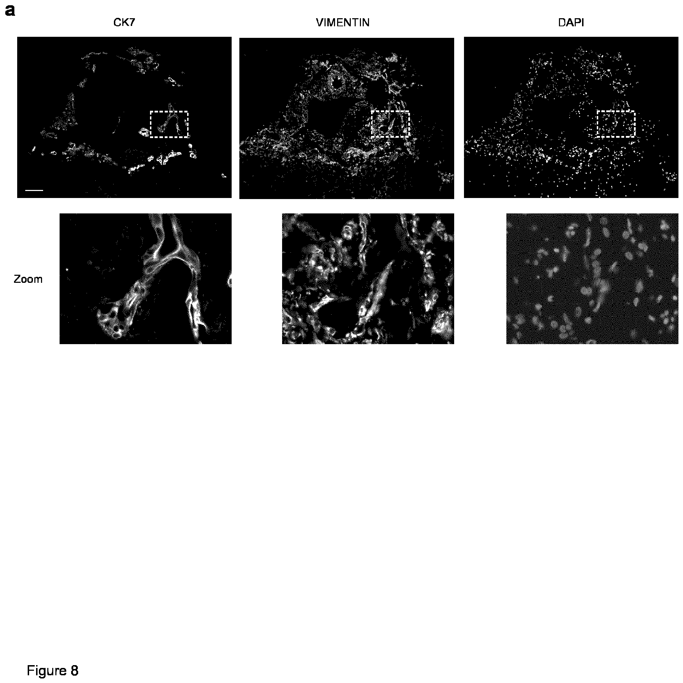

**Figure 8****:** Presence of mesenchymal cells in human PCLSs. Immunofluorescence of CK7 (left), vimentin (centre) and DAPI (right) in human PCLSs. Scale bar: 100 µm.

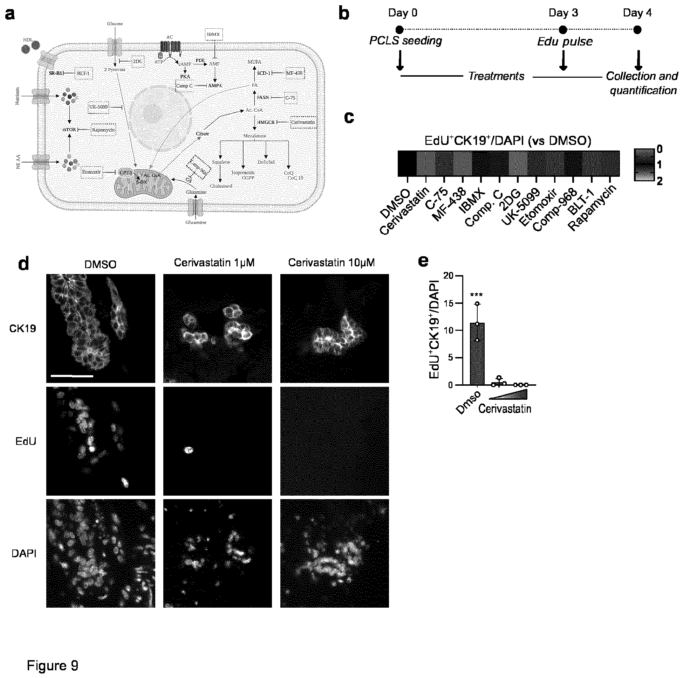

**Figure 9****:** screening of metabolic inhibitors. **a)** Schematic representation of main metabolic signalling pathways and selective inhibitors used in the screening. **b)** Time scheme of PCLS treatment with metabolic signalling pathway inhibitors. Proliferation was evaluated with the EdU assay. **c)** Evaluation of proliferation of stem cells positive for both CK19 and EdU after treatment with the inhibitors according to the procedure described in point b. **d)** Immunofluorescence of CK19 (above), EdU (centre) and DAPI (right) in PCLSs treated with increasing doses of cerivastatin. Scale bar: 50 µm. **e)** Quantitation of cells positive for both CK19 and EdU in PCLSs treated with cerivastatin (n=3). The data are presented as mean ± SD; ***P < 0.001, one-way ANOVA.

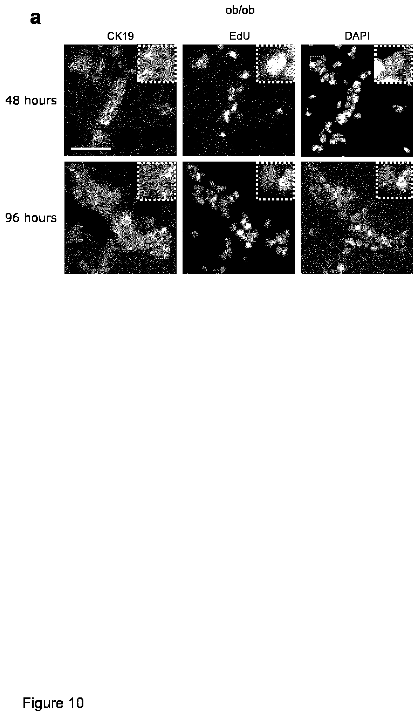

**Figure 10****:** proliferation of stem cells in an obesogenic context. **a)** Immunofluorescence of CK19 (left), EdU (centre) and DAPI (right) in PCLSs obtained from genetically obese mice (*ob*/*ob*)*.* Scale bar: 50 µm.

### DESCRIPTION OF THE INVENTION

The inventors have found a PCLS culture method that allows the generation and activation of bipotent liver stem cells in their native tissue context. This allows the study of the behaviour of liver stem cells in response to given genetic or physiopathological conditions or in response to medicaments, molecules or specific treatments.

The subject of the present invention is an *in vitro* method for generation of bipotent liver stem cells, comprising the following steps:
i) preparing a liver tissue section in the form of a PCLS (Precision-cut Liver Slice) in a culture medium consisting of William's E Medium with the addition of L-glutamine, glucose, HEPES and penicillin/streptomycin (hereinafter called "BWE" or "basal William's E Medium");
ii) transferring said PCLS into a culture medium comprising BWE, human or mouse EGF, and preferably one or more of the following ingredients: A83-01, B27 supplement; N-acetylcysteine; human gastrin 1; N2 supplement; human FGF 10; human HGF; RSPO1; nicotinamide; forskolin; noggin and/or Wnt3a;
iii) maintaining the PCLS in culture for a sufficient time to detect the generation of bipotent liver stem cells.

In a preferred embodiment, the PCLS derives from human liver, and the culture medium referred to in step ii) comprises: BWE medium; B27 supplement; N2 supplement; N-acetylcysteine; human gastrin 1; human EGF; human FGF10; human HGF; RSPO1; nicotinamide; forskolin; A83-01; noggin; and Wnt3a.

In a further preferred embodiment, the PCLS derives from mouse liver, and the culture medium referred to in step ii) comprises BWE medium; B27 supplement; N-acetylcysteine; human gastrin 1; mouse EGF; human FGF10; human HGF; RSPO1; and nicotinamide.

In a preferred, independent embodiment, the culture medium used in step ii) contains:
B27 supplement diluted 1:50; N-acetylcysteine 1 mM; human gastrin-1 10 nM; human EGF 50 ng/ml; human FGF10 100 ng/ml; human HGF 25 nM; RSPO1 1 µg/ml; nicotinamide 10 mM; N2 supplement diluted 1:100; forskolin 10 µM; A83-01 5 µM; noggin 25 ng/ml; and/or Wnt3a 50 ng/ml.

The method according to the invention offers a number of advantages over current techniques for *in vitro* stem cell study. Firstly, PCLSs maintain their complex multicellular architecture and the natural geometrical configuration of the liver tissue, including the organisation of the hepatocytes, the bile duct epithelial cells and the extracellular matrix. Moreover, PCLSs retain the entire cell ecosystem present in the liver tissue, including the hepatocytes, bile duct epithelial cells, immune cells, endothelial cells and stromal cells. Finally, PCLSs simulate the *in vivo* conditions and functional properties of the liver tissue unlike isolated stem cells, providing a more realistic representation of the liver physiology wherein the stem cells and the DR that generates them are involved.

In a preferred embodiment, in the method according to the invention the PCLSs derive from mouse or human liver.

The liver tissue sample selected for generation of PCLSs can be of mouse or human origin, such as samples deriving from biopsies or resections.

In a preferred aspect, in the method according to the invention the generation of the liver stem cells referred to in step iii) can be detected by an assay with EdU combined with immunofluorescence for the stem-cell markers.

The generation of the stem cells can be evaluated on the basis of their activation and/or proliferation. EdU (Click-iT^{®} EdU Cell Proliferation Kit, Life Technology) is added to the culture medium 24 hours before the samples are harvested. The stem-cell markers are preferably CK19 and CK7.

In one embodiment, the PCLS referred to in step (i) of the method according to the invention is obtained by:
a) providing a sample of liver tissue;
b) transferring said sample into a sterile basal culture medium comprising William's E Medium with the addition of 2 mM L-glutamine, 25 mM glucose, 10 mM HEPES, and 1% penicillin/streptomycin;
c) cutting said liver tissue into a cylinder with a diameter ranging between 5 and 7 mm;
d) embedding said cylinder in 4% agarose;
e) cutting the cylinder into sections (PCLSs) with a thickness ranging between 200 and 300 µm, preferably between 250 and 260 µm, and a diameter preferably ranging between 5 mm and 7 mm, using a vibratome;
f) transferring said PCLSs into the wells of a multi-well plate and into a culture in the same medium as described in point b);
g) incubating said PCLSs at 37C°, 5% CO2.

**In** one embodiment, said PCLS according to the invention has a thickness of 255 µm.

**In** a different embodiment, said PCLS according to the invention has a diameter of 6 mm.

**In** a preferred embodiment, said PCLS according to the invention has a thickness of 255 µm and a diameter of 6 mm.

The method according to the invention can include. after step (i) and before step (ii), the further step of encapsulating the PCLSs in an extracellular matrix of natural or artificial origin.

**In** a preferred aspect, said extracellular matrix is selected from a list consisting of a reconstituted basement membrane, in particular BME2 and matrigel; collagen I extracted from rat tail; and synthetic basement membrane based on the polymer 8arm polyethylene glycol (PEG) vinylsulphone (hexaglycerol), MW 40000.

The PCLS according to the invention is encapsulated between two layers of extracellular matrix extract, preferably an extract of Cultrex^{™} type 2 basement membrane diluted 1:3 in basal culture medium (BWE).

In a further aspect, the layer of said extracellular matrix below the PCLS is deposited on a polycarbonate insert, preferably with 8 µm pores.

Fixing of the matrix to the polycarbonate membrane is advantageous because both surfaces are exposed to the culture medium, allowing better diffusion of the nutrients in the innermost layers of the PCLS.

The method according to the invention can conveniently be applied to screening of medicaments and/or molecules able to modulate liver stem-cell generation, and for said purpose involves the following further steps:
- adding said medicament or molecule to the PCLS in culture in one of steps i) to iii) of the method for generation of bipotent liver stem cells according to the present invention;
- comparing the amount of stem cells generated in the presence of said medicament or molecule with those generated by performing the same method in the absence of the medicament or molecule,
whereby:
- increased generation of stem cells in the presence of the medicament or molecule indicates that said medicament or molecule is an activator of liver stem cells;
- decreased generation of stem cells in the presence of the medicament or molecule indicates that said medicament or molecule is an inhibitor of liver stem cells.

A further subject of the present invention is a culture medium for PCLS of human derivation, comprising BWE medium; B27 supplement; N2 supplement; N-acetylcysteine; human gastrin 1; human EGF; human FGF10; human HGF; RSPO1; nicotinamide; forskolin; A83-01; noggin; and Wnt3a.

A further subject of the present invention is a culture medium for PCLS of mouse derivation, comprising BWE medium; B27 supplement; N-acetylcysteine; human gastrin 1; mouse EGF; human FGF10; human HGF; RSPO1; and nicotinamide.

In a preferred embodiment, said media contain, independently of one another, B27 supplement diluted 1:50; N-acetylcysteine 1 mM; human gastrin 1 10 nM; human or mouse EGF 50 ng/ml; human FGF10 100 ng/ml; human HGF 25 nM; RSPO1 1 µg/ml; nicotinamide 10 mM; N2 supplement diluted 1:100; forskolin 10 µM; A83-01 5 µM; noggin 25 ng/ml; and/or Wnt3a 50 ng/ml.

The culture medium according to the invention is particularly suitable to stimulate liver stem-cell generation in liver tissue cultures, preferably to stimulate liver stem-cell generation in PCLS.

### EXAMPLES

### Protocol for generation of PCLS from liver tissue

The PCLSs are generated from mouse and human liver tissue. Immediately after removal, the liver samples are transferred into a sterile, cold basal culture medium (BWE) (William's E medium (Lonza, BE02019F), 2 mM L-glutamine (Euroclone, ECB3004D), 25 mM glucose, 10 mM HEPES (Sigma, H3375) and 1% penicillin/streptomycin, stored in ice until subsequent processing. The liver tissue is cut into 6 mm diameter cylinders with a dermal biopsy punch die and embedded in 4% agarose (Merck, Darmstadt, Germany). Tissue sections with a thickness of 250 µm were obtained with a VT1000S vibratome (Leica Biosystem, Wetzlar, Germany) and transferred into BWE in ice immediately after cutting. At the end of the cutting procedure, the PCLSs are transferred into BWE pre-heated to 37°C and incubated at 37°C, 5% CO2, for 2 hours' recovery. During the recovery period, the PCLSs are placed under stirring for 5 minutes at room temperature every 25 minutes. The PCLSs are then incorporated between two layers of Cultrex^{™} Type 2 basement membrane extract (R&D System, Minneapolis, USA) diluted 1:3 in BWE. The bottom layer is deposited on a polycarbonate insert with 8 µm pores (Corning, New York, USA) and left to solidify at 37°C. After 30 minutes, the PCLSs are gently positioned on the upper part of the matrix, covered with a layer of diluted BME (Cultrex^{™}), and incubated again at 37°C for 30 minutes. The PCLSs are then maintained in a 24-well plate, in EM medium (expansion medium). The PCLSs are maintained in culture at 37°C, 5% CO2, and the medium is replaced every 24 hours. The composition of the EM medium is described in **Table 1.** As control, the PCLSs are also cultured by the classic method, ie. maintained in suspension in transwells in BWE basal medium.

**Table 1**

| ***Basal medium (BWE)*** | | | | |
|---|---|---|---|---|
| William's E, 1% L-glutamine, 25 mM glucose, 10 mM HEPES, 1% pen/strep | | | | |
| ***Ingredient*** | ***Expansion medium (mouse)*** | ***Expansion medium (human)*** | ***Manufacturer*** | ***Code*** |
| Basal medium | | | | |
| B27 | 1:50 | 1:50 | Thermo Fischer | 12587010 |
| N2 | | 1:100 | Thermo Fischer | 17502048 |
| N-acetylcysteine | 1 mM | 1 mM | Sigma | A9165 |
| human gastrin 1 | 10 nM | 10 nM | Sigma | G9145 |
| mouse EGF | 50 ng/ml | | Pepro Tech | 315-09 |
| human EGF | | 50 ng/ml | Pepro Tech | AF-100-15 |
| human FGF10 | 100 ng/ml | 100 ng/ml | GenScript | z03314 |
| human HGF | 50 nM | 25 nM | Pepro Tech | 100-39H |
| RSPO1 | 1 µg/ml | 1 µg/ml | Pepro Tech | 120-38 |
| nicotinamide | 10 mM | 10 mM | Sigma | N0636 |
| forskolin | | 10 µM | BioGems | 6652995 |
| A83-01 | | 5 µM | BioGems | 9094360 |
| noggin | | 25 ng/ml | Pepro Tech | 120-10C |
| Wnt3a | | 50 ng/ml | HumanKine | HZ1296 |

### Protocol for monitoring generation of bipotent liver stem cells

The activation and proliferation capacity of the liver stem cells deriving from bile duct epithelium can be monitored in the PCLSs with an EdU assay combined with immunofluorescence for the stem-cell markers: CK19 or CK7. Specifically, EdU (Click-iT^{®} EdU Cell Proliferation Kit, Life Technology, C10637) is added to the culture medium 24 hours before harvesting of samples at the final concentration of 10 µM. When the experiment time is reached, the PCLSs are washed off the basement membrane, incubating the polycarbonate insert in cold PBS at 4°C for 10 minutes under stirring.

The PCLSs are then transferred into 10% formalin wherein they are fixed at room temperature for 24 hours. The fixed PCLSs are washed twice in PBS and dehydrated by incubation in increasing concentrations of ethanol (70%, 80%, 95%, 100%, 100%, 100%) for 30 minutes under stirring, and clarified twice in xylene for 30 minutes. After two incubations in liquid paraffin at 57-60°C for 30 minutes, the PCLSs are embedded in paraffin and stored at 4°C until subsequent processing.

The 5 µm sections of the paraffin-embedded PCLSs are cut with a rotary microtome (Leica RM2255) immediately after cooling the paraffin blocks at -20°C for 15 minutes. The resulting sections are laid in a bath thermostated at 37°C, transferred to microscope slides, and left to dry at 37°C overnight.

Edu detection combined with immunofluorescence requires removal of the paraffin from the tissue sections with two consecutive xylene baths for 10 minutes, and rehydration by incubating in decreasing concentrations of ethanol (100%, 100%, 95%, 70%, distilled water) for 5 minutes. The PCLSs are then incubated in citrate buffer pH 6 (10 mM tribasic sodium citrate, 0.05% Tween^{®} 20) at 98°C for 25 minutes to unmask the antigens of interest. After cooling at room temperature, the samples are washed twice consecutively in distilled water and PBS and incubated in blocking solution (1% BSA, 0.01% Triton X-100) for 1 hour. Incubation with the primary antibody for CK19 (abcam, ab133496) or CK7 (Santa Cruz Biotechnology, sc-23876) is conducted in a wet chamber at 4°C overnight.

The next day, the PCLSs are washed in PBS and incubated with the secondary antibody conjugated with Alexa Fluor^{®} 594 (Jackson Immunoresearch) diluted 1:200 in blocking solution for 2 hours at room temperature. After two washes in PBS, the PCLSs are incubated in the dark with the EdU detection solution, prepared as stated in the kit specifications, for 30 minutes at room temperature.

Nuclear staining is conducted by incubating the PCLSs with 4',6-diamidino-2-phenylindole dihydrochloride (DAPI, Sigma, D8417) 1 µg/mL for 10 minutes. After mounting the coverslip with the fluorescence mountant (Prolong^{®} gold antifade, Life Technology, P10144), the samples are observed under the microscope (DM200, Leica) for acquisition of the images (Leica DFC7000T camera). For each experimental condition, the images of 3 different biological replicates are acquired.

The images are then analysed with ImageJ software (version 1.54f) (DOI: https://doi.org/10.1038/nmeth.2019). Proliferation was calculated as the ratio between the area occupied by the EdU+ nuclei of the CK19+ or CK7+ cells and the total area of the nuclei of the CK19+ or CK7+ cells.

### Activation of stem cells in vitro

To evaluate the efficacy of this approach, PCLSs were prepared from wild-type (WT) mouse liver, and cultured in both BWE and EM on transwell inserts (**Figure 1A**, **b)**. The PCLS cultured for at least 96 hours gave rise to significant expansion of the liver stem cells (**Figure 1C**).

To test the proliferation of the stem cells, the presence of proliferation markers in the stem cells (CK19+) was monitored with an EdU test. After 96 hours, the PCLSs derived from WT mice exhibited numerous stem cells positive for the EdU proliferation marker, distributed around the portal area, thus confirming that a DR process had been induced *ex vivo* (**Figure 2a**). The activation and proliferation of the stem cells was only observed in the PCLSs cultured with the method presented herein, not with the classic PCLS culture method (**Figures 2a**, **2b**).

### Preservation of structural liver microenvironment

To evaluate whether DR in the PCLSs takes place in a native, physiologically significant, periportal extracellular matrix, collagen distribution was monitored by staining with Sirius Red (Sigma, 365548). As expected, collagen was concentrated in the periportal regions, forming distinctive ring-shaped structures, and its pattern and distribution throughout the tissue remained unchanged over time **(****Figure 4A****).** Subsequently, "second harmonic generation" (SHG) microscopy, a technique that directly displays fibrillar collagen, was used to evaluate whether the native fibrillar composition of collagen was maintained in the PCLSs. It was discovered that the fluorescent SHG signal generated by the collagen fibrils distributed around the portal areas was fully preserved after 96 hours **(****Figure 4B****).** To further characterise the mechanical properties of the portal regions in the PCLSs, the collagen-rich areas were scanned with atomic force microscopy (AFM), and it was discovered that the elasticity of the portal matrix was fully preserved as native, with no loss of rigidity over time **(****Figure 4c****).**

Finally, mesenchymal portal cells (vimentin+), known for their ability to regulate DR through both physical and paracrine interactions with the liver stem cells, were identified in the PCLSs **(****Figure 3****).** Taken as a whole, said findings demonstrate that PCLS cultures activate liver stem cells, *ex vivo,* in a short period of time, in a physiologically and histologically native environment.

### PCLSs of origin human activate liver stem cells ex vivo

To validate said findings in a human context, the PCLS cultures were adapted to human liver slices (**Figures 5A**, **5b**), cultured in human expansion medium (HEM, **Table 1).** Using Sirius Red staining and the SHG technique, it was confirmed that the geometrical configurations of the liver lobules were preserved over time, and that the architectural integrity of the collagen-enriched portal areas remains unchanged **(****Figure 6****).** Subsequently, immunofluorescence analysis over time demonstrated that CK7+ cells undergo a considerable morphological change from quiescent, polarised ductular cells to a migratory phenotype, infiltrating the surrounding tissue, typical of stem cells **(****Figure 7b****).** Once again, the EdU test exhibited a subpopulation of stem cells within the PCLSs which enters the cell cycle after 4 days in culture, and begins to proliferate actively (**Figure 7a**, **b)**. After 6 days in culture, a considerable increase in liver stem-cell proliferation was observed, particularly in the collagen-enriched portal areas **(****Figure 7c****).** Finally, it was confirmed that stem-cell activation takes place in portal areas rich in stromal cells, indicating a native microenvironment and dynamic interaction between the epithelial and stromal cells in the sections **(****Figure 8****).** Said results confirm the potential of PCLSs implemented as an *in vitro* method for human DR.

### Screening with medicaments or molecules known for their importance in various metabolic pathways

For this purpose, the PCLSs were treated for 72 hours with a small collection of molecules, well-known for their regulatory activity on various metabolic pathways **(****Figure 9a****),** and subjected to an EdU assay to evaluate the effect of the molecules on liver stem-cell activation **(****Figure 9b****).** Confirming the reliability of said approach, it was discovered that rapamycin, an inhibitor of the mTOR pathway, already known for controlling DR, slightly reduced stem-cell activation in PCLSs **(****Figure 9c****).** It was also discovered that inhibitors of the metabolic pathways important for lipid biosynthesis (namely cerivastatin, C75 and MF-438) reduced stem-cell activation in the PCLSs almost entirely, without affecting the morphology or viability of the bile ducts **(****Figure 9c****).** Cerivastatin, in particular, belongs to a group of medicaments called statins, used to lower cholesterol levels in patients with hypercholesterolaemia by means of competitive inhibition of hydroxy-3-methylglutaryl coenzyme-A reductase (HMGCR) **(****Figures 9d**, **9e)****.**

The same methodological approach can also be applied to other types of molecule: metabolites, signalling molecules, nutrients, physical conditions (e.g. oxygen levels, pressure and shear stress).

### Results on PCLSs derived from obese mice

To demonstrate that PCLSs can be used to study liver stem-cell activation under specific physiopathological conditions, the same protocol was applied to PCLSs derived from obese mice. In PCLSs derived from genetically obese mice (B6.Cg-Lepob/J, Charles River), liver stem-cell activation took place considerably earlier, and was already evident after 48 hours **(****Figure 10****).**

The same methodological approach can also be applied to other types of pathological condition, such mice with metabolic, viral, oncological or degenerative diseases.

## Claims

1. An *in vitro* method for the generation of bipotent liver stem cells comprising the following steps:
i) providing a section of liver tissue in PCLS (Precision-cut Liver Slice) form in a culture medium comprising William's E Medium with the addition of L-glutamine, glucose, HEPES and penicillin/streptomycin (BWE or "basal William's E Medium");
ii) transferring said PCLS into a culture medium comprising BWE, human or mouse EGF and preferably one or more of the following components: A83-01; B27 supplement; N-acetyl-cysteine; human gastrin 1, N2 supplement; human FGF 10; human HGF; RSPO1; nicotinamide; forskolin; noggin and/or Wnt3a;
iii) maintaining the PCLS in culture for a sufficient time to detect the generation of bipotent liver stem cells.

2. Method according to claim 1 wherein the PCLS is derived from mouse or human liver.

3. Method according to claims 1-2, wherein the PCLS is derived from human liver and the culture medium referred to in step ii) comprises BWE medium; B27 supplement; N2 supplement; N-acetylcysteine; human gastrin 1; human EGF; human FGF10; human HGF; RSPO1; nicotinamide; forskolin; A83-01; noggin; and Wnt3a.

4. Method according to claims 1-2, wherein the PCLS is derived from mouse liver and the culture medium referred to in step ii) comprises BWE medium; B27 supplement; N-acetylcysteine; human gastrin 1; mouse EGF; human FGF10; human HGF; RSPO1; and nicotinamide.

5. Method according to claims 1-4, wherein the generation of liver stem cells referred to in step iii) is detected by an EdU assay combined with immunofluorescence for stem-cell markers.

6. Method according to claim 5, wherein said stem-cell markers are CK19 and CK7.

7. Method according to claim 1, wherein said PCLS has a thickness ranging between 250 and 260 µm and a diameter ranging between 5 mm and 7 mm.

8. Method according to claim 1 comprising, after step (i) and before step (ii), the further step of encapsulating the PCLSs in an extracellular matrix of natural or artificial origin.

9. Method according to claim 8, wherein said extracellular matrix is selected from the group consisting of reconstituted basement membrane, in particular BME2 and matrigel; collagen I extracted from rat tail; and synthetic basement membrane based on the polymer 8arm polyethylene glycol (PEG) vinylsulphone (hexaglycerol), MW 40000.

10. Method according to claims 1-9, for screening medicaments and/or molecules able to modulate the generation of liver stem cells, comprising the following further steps:
- adding said medicament or molecule to the cultured PCLS in one of steps i) to iii) according to claim 1;
- comparing the amount of stem cells generated in the presence of said medicament or molecule with the amount of stem cells generated by carrying out the same method in the absence of the medicament or molecule,
whereby:
- increased generation of stem cells in the presence of the medicament or molecule indicates that said medicament or molecule is an activator of liver stem cells;
- decreased generation of stem cells in the presence of the medicament or molecule indicates that said medicament or molecule is an inhibitor of liver stem cells.

11. Use of a culture medium comprising BWE medium; B27 supplement; N2 supplement; N-acetylcysteine; human gastrin 1; human EGF; human FGF10; human HGF; RSPO1; nicotinamide; forskolin; A83-01; noggin; and Wnt3a, to stimulate the generation of liver stem cells in a culture of human-derived PCLS.

12. Use of a culture medium comprising BWE medium; B27 supplement; N-acetylcysteine; human gastrin 1; mouse EGF; human FGF10; human HGF; RSPO1; and nicotinamide, to stimulate the generation of liver stem cells in a culture of mouse-derived PCLS.
